(19) Europäisches Patentamt
European Patent Office
Office européen des brevets

(11) Veröffentlichungsnummer: **0 054 672**
**A1**

(12) # EUROPÄISCHE PATENTANMELDUNG

(21) Anmeldenummer: **81108664.4**

(22) Anmeldetag: **22.10.81**

(51) Int. Cl.³: **C 07 D 417/04**, A 61 K 31/445

(30) Priorität: **23.12.80 DE 3048663**

(43) Veröffentlichungstag der Anmeldung: **30.06.82**
**Patentblatt 82/26**

(84) Benannte Vertragsstaaten: **AT BE CH DE FR GB IT LI LU NL SE**

(71) Anmelder: **Grünenthal GmbH, Patentabteilung**
**Steinfeldstrasse 2, D-5190 Stolberg (DE)**

(72) Erfinder: **Graudums, Ivars, Dr., von-Werner-Strasse 31, D-5190 Stolberg (DE)**
Erfinder: **Friderichs, Elmar, Dr., Zehntweg 24, D-5190 Stolberg (DE)**

(54) **Neue Lactamverbindungen, diese enthaltende Arzneimittel und Verfahren zur Herstellung dieser Verbindungen und Arzneimittel.**

(57) Die Erfindung betrifft neue Lactamverbindungen, diese enthaltende Arzneimittel (insbesondere zur oralen oder rektalen Applikation) und Verfahren zur Herstellung dieser Verbindungen und Arzneimittel. Die neuen Verbindungen, die sedierend und zum Teil krampfhemmend sowie in höheren Dosierungen auch hypnonarkotisch wirken, entsprechen der allgemeinen Formel

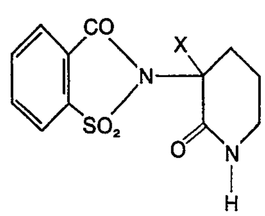

(I)

worin X ein Alkylrest mit 1 bis 4 Kohlenstoffatomen, insbesondere ein Methylrest ist. Die Herstellung der Verbindungen der Formel (I) erfolgt in an sich bekannter Weise, z.B. durch Umsetzung eines entsprechenden 3-Alkyl-3-halogen-2-oxopiperidins mit einer Metallverbindung des 2,3-Dihydro-3-oxo-1,2-benzisothiazol-dioxid-(1,1) oder durch Umsetzung des entsprechenden 3-Alkyl-3-amino-2-oxopiperidins mit dem Dichlorid oder Dibromid der o-Sulfobenzoesäure oder mit einem o-Halogenmercapto-benzoylhalogenid, gefolgt von einer Oxydationsreaktion. Andere an sich bekannte Reaktionen, wie z.B. Ringschlußreaktionen zur Bildung des Lactamringes, sind ebenfalls zur Herstellung der Verbindungen der Formel (I) geeignet.

EP 0 054 672 A1

ACTORUM AG

Patentanmeldung der Grünenthal GmbH, Stolberg/Rhld., Bundesrepublik Deutschland

Neue Lactamverbindungen, diese enthaltende Arzneimittel und Verfahren zur Herstellung dieser Verbindungen und Arzneimittel.

Die vorliegende Erfindung bezieht sich auf neue Lactamverbindungen mit einem breiten Spektrum von das Zentralnervensystem dämpfenden Wirkungen. Die Verbindungen entsprechen der allgemeinen Formel

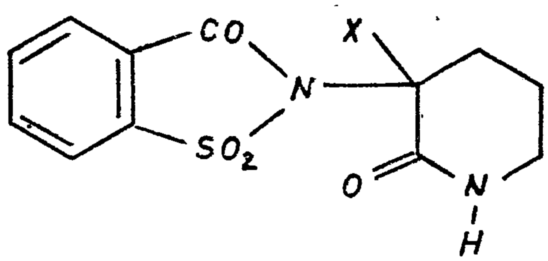

(I)

worin X für einen gegebenenfalls verzweigten Alkylrest mit 1 - 4 Kohlenstoffatomen steht.

Vorzugsweise bedeutet X einen Alkylrest mit ein oder zwei Kohlenstoffatomen, insbesondere aber den Methylrest.

Die Verbindungen der Formel I, insbesondere das 3-(2,3-Dihydro-1,1-dioxido-3-oxo-1,2-benzisothiazol-2-yl)-3-methyl-2-oxo-piperidin (nachstehend als "Methylverbindung" bezeichnet) haben sedierend-tranquilisierende Eigenschaften. In höheren Dosierungen wirken die Verbindungen, in denen X für Methyl oder Äthyl steht, auch hypno-narkotisch, wobei diese Wirkungstypen bei den erfindungsgemäßen Verbindungen (im Gegensatz zu den Verhältnissen bei Barbituraten und verwandten Verbindungen) durch einen großen Dosisabstand getrennt sind. Am Magendarmtrakt wirken die erfindungsgemäßen Verbindungen hemmend auf Sekretion und Motilität. Darüberhinaus zeichnet sich die Methylverbindung durch gute krampfhemmende Wirkungen aus.

Während nach Verabreichung von Barbituraten oder von Benzodiazepinen bei verschiedenen Tierspezies oder auch bei bestimmten Personen (z. B. geriatrischen Patienten) paradoxe Erregungszustände auftreten bzw. auftreten können, fehlt im Wirkungsspektrum der erfindungsgemäßen Verbindungen die erregende Wirkkomponente.

Die Verbindungen der allgemeinen Formel I, insbesondere die Methylverbindung, sind also zur Therapie und Prophylaxe von Unruhezuständen, psychomotorischer Erregung, Angstzuständen, Schlafstörungen, cerebralen Krampfleiden aus dem Formenkreis der Grand-mal- und Petit-mal-Epilepsien sowie von Hypermotilitätszuständen im Gastrointestinal-Bereich (Reizmagen, irritables Colon) indiziert.

Bei den genannten Indikationen können sie sowohl in der Humanmedizin als auch im Veterinärbereich eingesetzt werden.

Die Substanzen eignen sich insbesondere zur oralen oder rektalen Anwendung in den üblichen Zubereitungsformen. Für die orale Verabreichung kommen dementsprechend sowohl feste Formen, wie Tabletten, Dragees oder Kapseln in Betracht, wobei die Wirkstoffe gegebenenfalls mit üblichen Trägermaterialien,

Tablettensprengmitteln, Bindemitteln usw. vermischt bzw. verarbeitet sind, oder auch flüssige Formen, wie Sirupe, Säfte oder Tropfen. Insbesondere die festen oralen Verabreichungsformen können auch in üblicher Weise so hergestellt werden, daß sie den Wirkstoff verzögert freisetzen, um so über einen längeren Zeitraum eine möglichst gleichförmige Versorgung des Patienten mit dem Wirkstoff zu ermöglichen. Der Wirkstoffgehalt pro Einzeldosis soll etwa 50 bis 500 mg betragen, wobei für Depotzubereitungen entsprechend der Freigabekinetik erhöhte Wirkstoffkonzentrationen verwendbar sind. Die Tagesgesamtdosis beträgt je nach Schwere des Krankheitsbildes z. B. 50 bis 1000 mg, jedoch können dank der geringen Toxizität der Verbindungen der Formel I auch höhere Tagesgesamtdosen angewendet werden.

Aus der deutschen Patentschrift 2 210 166 und entsprechenden Schutzrechten in anderen Ländern sind Verbindungen der Formel

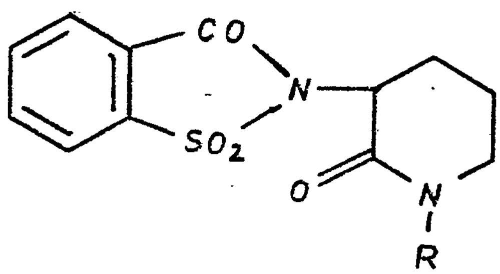

(A)

worin R für ein Wasserstoffatom oder einen Alkylrest mit 1 bis 3 Kohlenstoffatomen steht,

bekannt, die sich u. a. als Mittel zur Sedierung und als Hypnotika für Mensch und Tier eignen, denen aber keine narkotische Wirkung zukommt. (Z. B. konnte für die Verbindung der Formel A, in der R gleich Wasserstoff ist, im Tierexperiment auch bei intraperitonealer Gabe der extrem hohen Dosis von 10 000 mg/kg keine Narkosewirkung beobachtet werden.)

Demgegenüber haben die Verbindungen der Formel I, in denen X für Methyl oder Äthyl steht, wie bereits gesagt, bei höheren Dosierungen auch hypnonarkotische Eigenschaften. So wurden als Werte der $ED_{50}$ (d. h. derjenigen Dosis, deren Verabreichung bei 50 % der Tiere den betreffenden Effekt bewirkt) für die "Methylverbindung" hinsichtlich der sedativen Wirkungen 33,3 mg/kg (intraperitoneal) bzw. 258 mg/kg (oral) ermittelt, während der $ED_{50}$-Wert für die narkotischen Wirkungen bei 592 mg/kg (intraperitoneal) liegt.

Im Vergleich zu den Verbindungen der Formel (A) konnte für die Verbindung der Formel I ($X = CH_3$) auch eine Erweiterung der antikonvulsiven Wirksamkeit gefunden werden. Während z. B. die Verbindung A (R = H) nur gegenüber experimenteller Krampfauslösung durch den Elektroschock ($ED_{50}$ 444 mg/kg i.p.) wirkt, also dem Wirktyp des Diphenylhydantoins entspricht, ist die "Methylverbindung" im gleichen Dosisbereich wie gegen den durch den Elektroschock ausgelösten Krampf ($ED_{50}$ 112 mg/kg i.p.) auch gegenüber dem Pentetrazol-Klonus ($ED_{50}$ 91,4 mg/kg i.p.) wirksam, entspricht also zusätzlich noch dem Wirktyp des Phenobarbitals in dieser Indikation. Dabei ist noch besonders zu erwähnen, daß für die vorbekannte Verbindung A (R = H) die beschriebene Hemmung des durch Elektroschock ausgelösten Krampfes nur bei intraperitonealer, nicht aber bei oraler Verabreichung nachweisbar ist, während für die hier beschriebene "Methylverbindung" bei oraler Gabe die $ED_{50}$ 181 mg/kg beträgt. Diese wertvollen Erweiterungen des Wirkungsspektrums bei den Verbindungen der allgemeinen Formel I, insbesondere bei der Methylverbindung, im Vergleich zu dem der vorbekannten Substanzen waren überraschend und unvorhersehbar.

Die Herstellung der neuen wertvollen Verbindungen der allgemeinen Formel I erfolgt durch Umsetzung einer Verbindung der Formel

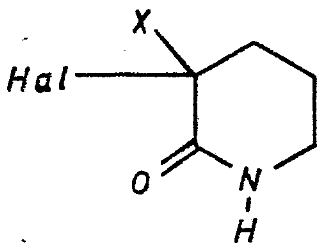

(II)

in der X die gleiche Bedeutung wie oben hat und Hal für ein Jodatom oder vorzugsweise für ein Chlor- oder Bromatom steht,

mit einer Verbindung der allgemeinen Formel

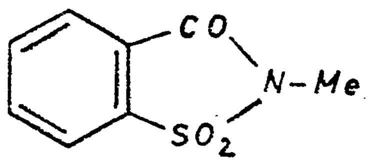

(III)

worin Me für Alkalimetall, vorzugsweise Natrium oder Kalium, steht.

Die Reaktion wird im allgemeinen so durchgeführt, daß man die Verbindung der allgemeinen Formel II in einem geeigneten Lösungsmittel auflöst und mit einer Suspension oder Lösung der Verbindung der allgemeinen Formel III, gegebenenfalls unter Zusatz von etwas Natrium- oder Kaliumjodid, vereinigt. Geeignete Reaktionsmedien sind z. B. Dimethyl-formamid oder -acetamid, Dimethylsulfoxid, aliphatische Alkohole mit 1 - 8 Kohlenstoffatomen, Butylacetat, Acetonitril, offenkettige oder cyclische Äther wie Tetrahydrofuran oder Dioxan, Toluol, Xylol, Cyclohexan, n-Heptan und ähnliche Lösungs- oder Suspensionsmittel. Die Reaktion erfolgt vorzugsweise bei der Siedetemperatur des verwendeten Mediums, sie kann jedoch bei entsprechender Verlängerung der Reaktionszeit auch bei Raumtemperatur oder unter Kühlung durchgeführt

werden. Zur Aufarbeitung der Reaktionslösung kann man mit Wasser versetzen und mit geeigneten organischen Lösungsmitteln wie Äther, Kohlenwasserstoffen, Halogenkohlenwasserstoffen usw. extrahieren, man kann jedoch auch das verwendete Lösungsmittel abdestillieren und das als Rückstand verbleibende Reaktionsprodukt mit Wasser waschen und durch Umkristallisieren reinigen.

Die Verbindungen der allgemeinen Formel I können auch hergestellt werden, indem man eine Verbindung der allgemeinen Formel

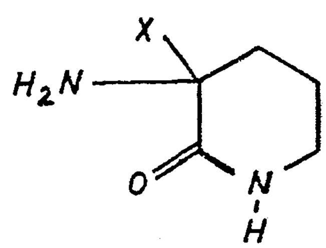

(IV)

worin X die gleiche Bedeutung wie oben hat

oder ein Salz einer Verbindung der allgemeinen Formel IV mit o-Sulfobenzoesäuredichlorid oder -dibromid umsetzt. Als Salze der Verbindungen der Formel IV kommen dabei z. B. die Hydrochloride, Hydrobromide, Sulfate, Phosphate, Formiate, Acetate, Benzolsulfonate und ähnliche Salze mit Säuren in Betracht.

Die Reaktion wird zweckmäßig in indifferenten Lösungsmitteln, gegebenenfalls unter Kühlung und Zusatz von säurebindenden Stoffen, wie Triäthylamin, Trimethylamin, Diisopropyläthylamin, Natriumcarbonat, Kaliumcarbonat, Natriumhydrogencarbonat oder Kaliumhydrogencarbonat usw. durchgeführt. Als Lösungsmittel kommen mit Ausnahme der Alkohole die gleichen Stoffe wie die für die Umsetzung der Verbindungen II und III genannten in Betracht, jedoch werden hier Dimethylformamid,

die Äther und Kohlenwasserstoffe bevorzugt. Die Aufarbeitung des Reaktionsgemisches erfolgt in gleicher Weise wie für den Fall der Umsetzung der Verbindungen II und III oben beschrieben.

Man kann die Verbindungen der Formel I aus den Verbindungen der Formel IV auch dadurch erhalten, daß man die Verbindung der Formel IV mit einer Verbindung der Formel

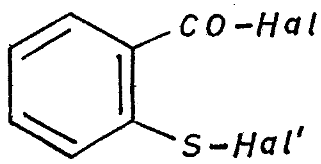

(V)

worin Hal und Hal' gleich oder verschieden sind und ein Brom- oder Jodatom oder vorzugsweise ein Chloratom bedeuten

in Gegenwart eines Lösungs- oder Suspensionsmittels, zweckmäßig in Gegenwart einer Base bei Temperaturen zwischen etwa $0^{\circ}$ und $100^{\circ}C$ umsetzt und dann die so erhaltene Verbindung der Formel

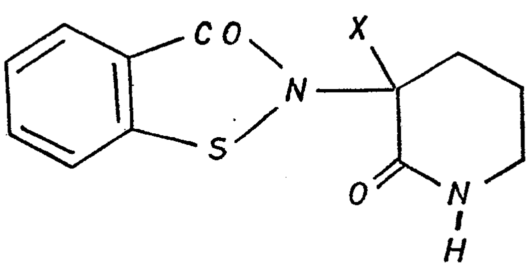

(VI)

worin X die gleiche Bedeutung wie oben hat,

in Gegenwart einer aliphatischen Carbonsäure mit 1 bis 5 Kohlenstoffatomen als Lösungsmittel bei Temperaturen zwischen etwa $25^{\circ}$ und $125^{\circ}C$ oxydiert. Als Oxydationsmittel kommen insbesondere Wasserstoffperoxid oder Persäuren wie Peroxyessigsäure, Peroxypropionsäure, Peroxybenzoesäure usw. in Betracht,

jedoch können auch Oxydationsmittel wie Kaliumpermanganat Anwendung finden.

Geeignete, bei der Reaktion der Verbindung der Formel IV mit der Verbindung der Formel V einzusetzende Basen sind z. B. tertiäre Amine wie Triäthylamin, Tripropylamin, N-Äthylpiperidin oder insbesondere Pyridin oder ein Picolin, die zugleich als Lösungsmittel dienen können.

Die Aminopiperidinone-(2) der allgemeinen Formel IV sind z. T. bereits aus der Literatur bekannt. Sie können leicht aus den Benzaliminopiperidonen der allgemeinen Formel

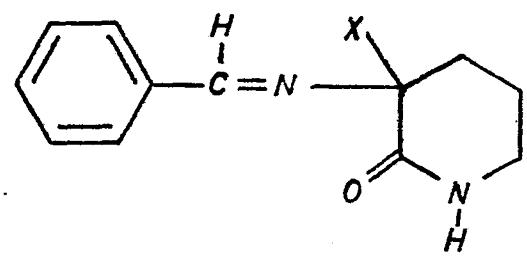

worin X die gleiche Bedeutung wie oben hat,

dadurch erhalten werden, daß man diese in einem indifferenten Lösungsmittel, z. B. Alkohol, bei Gegenwart von Edelmetallkatalysatoren, vorzugsweise Palladiumkohle, zweckmäßig bei 20 - 40°C und bei Normaldruck hydriert, obwohl man auch erhöhte Temperaturen und Wasserstoffdrucke verwenden kann. Die Aufarbeitung erfolgt in der Weise, daß man vom Katalysator abfiltriert und die Lösung einengt. Die Verbindungen der Formel IV entstehen dabei im allgemeinen in für die weitere Umsetzung genügender Reinheit.

Die Verbindungen der Formel V sind ebenfalls bereits bekannt. Sie lassen sich beispielsweise ausgehend vom Diphenyldisulfid-2,2'-dicarbonsäuredichlorid oder -dibromid durch Halogenierung, insbesondere durch Behandlung mit Chlor oder Brom in

Gegenwart eines Lösungsmittels wie Dichlormethan, Chloroform oder Tetrachlorkohlenstoff erhalten.

Die Verbindungen der allgemeinen Formel I können ferner hergestellt werden, indem man eine Verbindung der allgemeinen Formel

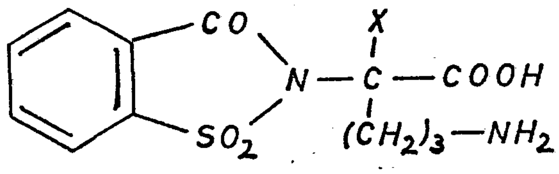

(VII),

worin X die gleiche Bedeutung wie oben hat,

unter Wasserabspaltung cyclisiert. Der Ringschluß kann unter Verwendung von Phosphorpentachlorid, Thionylchlorid, Acetylchlorid, Chlorwasserstoff oder ähnlichen sauren, die Wasserabspaltung fördernden Verbindungen erfolgen. Er kann aber auch alleine durch Erhitzen, gegebenenfalls in Gegenwart eines hoch siedenden Lösungs- oder Suspensionsmittels, bewirkt werden. Gegebenenfalls kann man auch ein funktionelles Derivat der Säure der allgemeinen Formel VII, wie z. B. ein Säurehalogenid oder einen Ester verwenden und dann den Ringschluß unter Abspaltung des entsprechenden Halogenwasserstoffes oder Alkohols bewirken.

Weiterhin kann die Herstellung der Verbindungen der allgemeinen Formel I dadurch erfolgen, daß man eine Verbindung der allgemeinen Formel

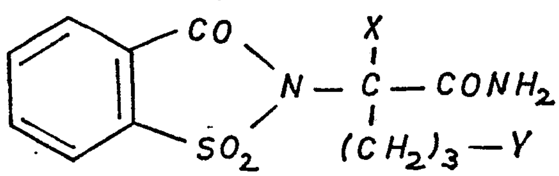

(VIII).

worin X die gleiche Bedeutung wie oben hat und Y für ein Chlor-, Brom- oder Jodatom oder für eine Hydroxylgruppe steht,

unter Abspaltung der Verbindung HY (also Chlor-, Brom- oder Jodwasserstoff bzw. Wasser) cyclisiert. Dabei arbeitet man, wenn Y eines der genannten Halogenatome darstellt, in Gegenwart von anorganischen oder organischen Basen. Als solche können z. B. Ammoniak, wässrige Kali- oder Natronlauge, Alkalialkoholate oder Amine, beispielsweise Triäthylamin, verwendet werden. Hierbei kann das Amin zusätzlich als Lösungsmittel dienen, man kann jedoch auch Lösungsmittel wie Benzol, Toluol oder Alkohole verwenden. Die Reaktion verläuft sowohl bei Raumtemperatur als auch bei erhöhter Temperatur.

Steht dagegen Y für die Hydroxylgruppe, so führt man die Ringschlußreaktion vorzugsweise unter Zusatz saurer, wasserabspaltend wirkender Mittel, wie z. B. Thionylchlorid, Acetylchlorid, Acetanhydrid, die auch als Lösungs- bzw. Suspensionsmittel dienen können, gegebenenfalls in Gegenwart eines indifferenten Lösungs- oder Suspensionsmittels, durch. Der Ringschluß kann aber auch allein durch Erhitzen, gegebenenfalls in einem hochsiedenden Lösungs- oder Suspensionsmittel, bewirkt werden.

Nach Durchführung der Ringschlußreaktion erfolgt die Aufarbeitung des Reaktionsgemisches wie bei den vorstehenden Verfahrensweisen beschrieben.

Die Verbindungen der allgemeinen Formel I enthalten ein asymmetrisch substituiertes Kohlenstoffatom und können daher in optisch aktiven Formen auftreten. Die Erfindung umfaßt sowohl die Racemate als auch die optisch aktiven Formen der Verbindungen der Formel I bzw. ihre Herstellungsverfahren.

Zur Herstellung der optisch aktiven Formen geht man zweckmäßig von optisch aktiven Formen der Verbindungen der Formeln II bzw. IV aus und setzt diese, wie oben beschrieben, mit einer Verbindung der Formel III bzw. mit o-Sulfobenzoesäuredichlorid oder -dibromid bzw. mit einer Verbindung der Formel V, gefolgt von einer Oxydationsreaktion, um. Man kann aber auch bei den weiteren oben beschriebenen Verfahrensvarianten von optisch aktiven Verbindungen ausgehen oder die Razemate der Verbindungen der Formel I auftrennen.

Die folgenden Beispiele dienen zur weiteren Erläuterung der Erfindung. Alle Temperaturangaben sind unkorrigiert. Bei der Durchführung der Beispiele wurde nicht auf Erzielung maximaler Ausbeuten hingearbeitet.

Beispiel 1

6,8 g 3-Brom-3-methylpiperidinon-(2) und 8 g getrocknetes Natriumsalz des 2,3-Dihydro-3-oxo-1,2-benzisothiazol-dioxid-(1,1) werden in 25 ml destilliertem Dimethylformamid 75 Minuten unter Rühren auf 110 - 115$^{o}$C erhitzt. Man kühlt ab, gibt 75 ml Wasser hinzu und rührt 30 Minuten nach. Dann saugt man vom ausgefallenen Niederschlag ab, wäscht mit Wasser und trocknet bei 80$^{o}$C im Vakuum. Durch Umkristallisieren aus n-Butanol erhält man das 3-(2,3-Dihydro-1,1-dioxido-3-oxo-1,2-benzisothiazol-2-yl)-3-methyl-2-oxo-piperidin vom Schmelzpunkt 211 - 215$^{o}$C. Ausbeute 24 % der Theorie.

Das in vorstehendem Beispiel als Ausgangsmaterial verwendete 3-Brom-3-methylpiperidinon-(2) erhält man auf folgende Weise:

5,15 g 3-Methylpiperidinon-(2) (Ber. 24, S. 2445) und 50 ml Chloroform werden auf 0$^{o}$C abgekühlt. Nun trägt man unter Rühren bei 0 - 5$^{o}$C 19 g Phosphorpentachlorid ein, gibt 0,2 g

wasserfreies Zinkchlorid hinzu, erwärmt auf 15°C und läßt 2,5 ml Brom in 5 ml Chloroform zutropfen. Man erwärmt unter Rühren 5 Stunden auf 40°C. Den abgekühlten Ansatz gibt man auf Eis und versetzt bis zur Entfärbung mit festem Natriumhydrogensulfit. Nun wird die Chloroformschicht abgetrennt und die wässrige Phase mit Chloroform extrahiert. Die vereinigten Chloroformlösungen trocknet man über Magnesiumsulfat und destilliert das Chloroform im Vakuum ab. Es entsteht ein halbfestes Produkt, das aus Aceton umkristallisiert wird. Man erhält so das 3-Brom-3-methylpiperidinon-(2) in einer Ausbeute von 81 % der Theorie. Schmelzpunkt 107 - 109°C.

Beispiel 2

6,3 g 3-Amino-3-methyl-piperidinon-(2) [J.Med.Chem. 21, 50 - 55 (1978)] werden in 46 ml absolutem Dimethylformamid gelöst. Unter Eiskühlung gibt man 11,8 g o-Sulfobenzoesäuredichlorid vom Schmelzpunkt 79°C (Beilstein, Hauptwerk, Band 11, 374) hinzu und gibt anschließend tropfenweise bei 0°C 13,9 ml Triäthylamin zu dem Reaktionsgemisch. Man rührt 24 Stunden bei 0°C, gibt dann unter Eiskühlung 120 ml destilliertes Wasser hinzu, extrahiert mit Chloroform und wäscht die Chloroformlösung mit Wasser. Die über Magnesiumsulfat getrocknete Chloroformlösung wird dann im Vakuum eingeengt. Den Rückstand löst man in wenig absolutem Äthanol, rührt im Eisbad, saugt die entstandenen Kristalle ab und kristallisiert aus n-Butanol um. Man erhält so das 3-(2,3-Dihydro-1,1-dioxido-3-oxo-1,2-benzisothiazol-2-yl)-3-methyl-2-oxopiperidin in Form weißer Kristalle vom Schmelzpunkt 211 - 215°C, identisch mit dem nach Beispiel 1 erhaltenen Produkt. Ausbeute 64 % der Theorie.

Beispiel 3

2,05 g 3-Äthyl-3-aminopiperidinon-(2) werden in 15 ml absolutem Dimethylformamid gelöst. Unter Eiskühlung gibt man 3,45 g o-Sulfobenzoesäuredichlorid hinzu und gibt anschliessend tropfenweise bei 0 - 5$^{o}$C 4,05 ml Triäthylamin zu dem Reaktionsgemisch. Man läßt einige Zeit unter Eiskühlung nachreagieren, hält anschließend einige Stunden bei Raumtemperatur, gibt dann unter Eiskühlung 45 ml destilliertes Wasser hinzu, extrahiert mit Chloroform und wäscht die Chloroformlösung mit Wasser. Die über Magnesiumsulfat getrocknete Chloroformlösung wird dann im Vakuum eingeengt. Den Rückstand löst man in heißem Essigsäureäthylester, läßt abkühlen, saugt die entstandenen Kristalle ab und kristallisiert aus n-Butanol um. Man erhält so das 3-Äthyl-3-(2,3-dihydro-1,1-dioxido-3-oxo-1,2-benzisothiazol-2-yl)-2-oxopiperidin in Form weißer Kristalle vom Schmelzpunkt 207 - 209$^{o}$C in einer Ausbeute von 45 % der Theorie.

Das in vorstehendem Beispiel als Ausgangsmaterial verwendete 3-Äthyl-3-aminopiperidinon-(2) erhält man auf folgende Weise:

3,3 g 3-Äthyl-3-benzaliminopiperidinon-(2) [J.Med.Chem. 18, 600 ff (1975)] werden in 200 ml absolutem Äthanol gelöst. Man gibt 5 g 10 %-ige Palladiumkohle hinzu und hydriert bei Raumtemperatur und Normaldruck, bis die theoretisch berechnete Menge Wasserstoff aufgenommen ist. Nun saugt man vom Katalysator ab, destilliert den Äthanol im Vakuum ab und trocknet den festen Rückstand im Vakuum bei 50$^{o}$C. Man erhält so das 3-Äthyl-3-aminopiperidinon-(2) in einer Ausbeute von 95 % der Theorie. Das Produkt ist für die weitere Umsetzung genügend rein.

Beispiel 4

5 g 5-Amino-2-(2,3-dihydro-1,1-dioxido-3-oxo-1,2-benzisothiazol-2-yl)-2-methyl-valeriansäure werden in 150 ml Toluol am Wasserabscheider bis zur Beendigung der Wasserabspaltung gekocht. Nach dem Abkühlen wird im Vakuum auf ein kleines Volumen eingeengt und anschließend filtriert. Der Rückstand wird aus n-Butanol umkristallisiert. Man erhält so das 3-(2,3-Dihydro-1,1-dioxido-3-oxo-1,2-benzisothiazol-2-yl)-3-methyl-2-oxopiperidin vom Schmelzpunkt 211 - 215°C, identisch mit dem nach Beispiel 1 erhaltenen Produkt. Ausbeute 31 % der Theorie.

Beispiel 5

3,75 g 5-Brom-2-(2,3-dihydro-1,1-dioxido-3-oxo-1,2-benzisothiazol-2-yl)-2-methyl-valeriansäureamid werden in 100 ml Xylol gelöst. Unter Zusatz von 1,3 g Äthyldiisopropylamin erhitzt man 2 Stunden auf Rückflußtemperatur. Nach dem Abkühlen wird im Vacuum auf ein kleines Volumen eingeengt und anschließend filtriert. Der Rückstand wird umkristallisiert aus n-Butanol. Man erhält das 3-(2,3-Dihydro-1,1-dioxido-3-oxo-1,2-benzisothiazol-2-yl)-3-methyl-2-oxopiperidin vom Schmelzpunkt 211 - 215°C, identisch mit dem nach Beispiel 1 erhaltenen Produkt. Ausbeute 25 % der Theorie.

Beispiel 6

5 g 2-(2,3-Dihydro-1,1-dioxido-3-oxo-1,2-benzisothiazol-2-yl)-5-hydroxy-2-methyl-valeriansäureamid werden in 150 ml Xylol gelöst und am Wasserabscheider mehrere Stunden auf Rückflußtemperatur erhitzt. Nach Abscheidung der theoretischen Wassermenge läßt man abkühlen, engt im Vacuum auf ein kleines Volumen ein, filtriert und erhält so das 3-(2,3-Dihydro-1,1-dioxido-3-oxo-1,2-benzisothiazol-2-yl)-3-methyl-2-oxopiperidin in Form weißer Kristalle. Nach Umkristallisieren aus n-Butanol schmilzt die Verbindung bei 211 - 215°C und ist identisch mit dem nach Beispiel 1 erhaltenen Produkt. Ausbeute 37 % der Theorie.

Beispiel 7

Man verfährt wie in Beispiel 2, verwendet aber statt des 3-Amino-3-methyl-piperidinon-(2) 7,7 g 3-Amino-3-n-propyl-piperidinon-(2) bzw. 8,4 g 3-Amino-3-n-butyl-piperidinon-(2) und erhält so:

3-(2,3-Dihydro-1,1-dioxido-3-oxo-1,2-benzisothiazol-2-yl)-3-n-propyl-2-oxopiperidin vom Schmelzpunkt 235 - 237°C in einer Ausbeute von 26 % der Theorie bzw.

3-n-Butyl-3-(2,3-dihydro-1,1-dioxido-3-oxo-1,2-benzisothiazol-2-yl)-2-oxopiperidin vom Schmelzpunkt 220 - 222°C in einer Ausbeute von 21 % der Theorie.

Beispiel 8

11,8 g o-Sulfobenzoesäuredichlorid werden in 50 ml absolutem Tetrahydrofuran gelöst. Unter Eiskühlung und Rühren gibt man bei 0°C tropfenweise eine Lösung von 6,3 g 3-Amino-3-methyl-piperidinon-(2) und 13,9 ml Triäthylamin in 30 ml absolutem Dimethylformamid zu, rührt noch 24 Stunden bei 0°C und gibt dann unter Eiskühlung 250 ml Wasser zu. Das Gemisch wird filtriert, der Rückstand mit Wasser gewaschen und aus n-Butanol umkristallisiert, wobei man das gleiche Produkt wie in Beispiel 1 in einer Ausbeute von 68 % der Theorie erhält.

Beispiel 9

25,6 g 3-Amino-3-methyl-piperidinon-(2) werden bei -20°C in 300 ml absolutes Dimethylformamid eingetragen. Unter Rühren versetzt man mit 47,8 g o-Sulfobenzoesäuredichlorid, rührt für 15 Minuten und gibt dann tropfenweise bei -18° bis -20°C 55,2 ml Triäthylamin zu. Man rührt 45 Stunden bei -20°C und versetzt dann unter Kühlung langsam mit 400 ml Wasser, wobei man die Temperatur nicht über 0°C ansteigen läßt. Nach Zugabe von 200 ml Chloroform läßt man die Temperatur unter Rühren auf 18°C ansteigen und filtriert dann den entstandenen Niederschlag ab. Dieser wird mit Wasser gewaschen und nach Trocknen aus Methanol umkristallisiert, wobei man das 3-(2,3-Dihydro-1,1-dioxido-3-oxo-1,2-benzisothiazol-2-yl)-3-methyl-2-oxopiperidin in Form weißer Kristalle vom Schmelzpunkt 211° - 215°C in einer Ausbeute von 65 % der Theorie erhält.

Beispiel 10

4,3 g 3-Amino-3-methyl-piperidinon-(2) werden in 25 ml Pyridin gelöst und unter Rühren langsam mit einer Lösung von 6,9 g 2-Chlormercaptobenzoylchlorid in 25 ml Tetrachlorkohlenstoff versetzt. Man gibt weitere 25 ml Tetrachlorkohlenstoff zu, erhitzt unter Rühren für 30 Minuten auf 60°C, kühlt ab und versetzt dann mit Eiswasser. Nach Trennen der Schichten wird die wässrige Schicht mit Tetrachlorkohlenstoff extrahiert und die vereinigten organischen Lösungen werden mit Wasser gewaschen. Nach Trocknen über Magnesiumsulfat wird der Tetrachlorkohlenstoff im Vacuum abdestilliert. Der Rückstand wird in 25 ml Eisessig gelöst und unter Rühren langsam mit 10 ml einer 50 %-igen Lösung von Peroxyessigsäure in Essigsäure versetzt. Das Gemisch wird unter Rühren langsam auf 100°C erhitzt und nach 30 Minuten bei dieser Temperatur abgekühlt. Man gibt langsam 5 ml Methanol und nach 10 - 15 Minuten 200 ml Wasser zu. Der Niederschlag wird abfiltriert und aus n-Butanol umkristallisiert, wobei man das 3-(2,3-Dihydro-1,1-dioxido-3-oxo-1,2-benzisothiazol-2-yl)-3-methyl-2-oxo-piperidin in einer Ausbeute von 21 % der Theorie erhält.
Schmelzpunkt: 211 - 215°C.

Patentansprüche

1) Neue Lactamverbindungen der Formel

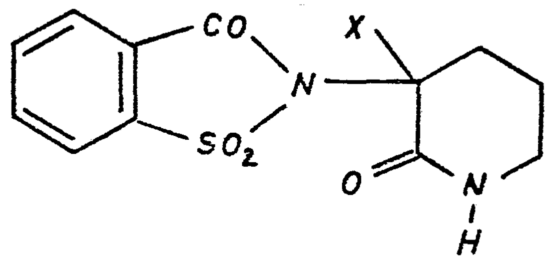

(I)

worin X für einen gegebenenfalls verzweigten Alkylrest mit 1 bis 4 Kohlenstoffatomen steht.

2) Neue Lactamverbindungen der Formel I gemäß Anspruch 1, dadurch gekennzeichnet, daß X für einen geradkettigen Alkylrest mit ein oder zwei Kohlenstoffatomen steht.

3) Als neue Lactamverbindung der Formel I gemäß Ansprüchen 1 und 2 das 3-(2,3-Dihydro-1,1-dioxido-3-oxo-1,2-benzisothiazol-2-yl)-3-methyl-2-oxopiperidin der Formel

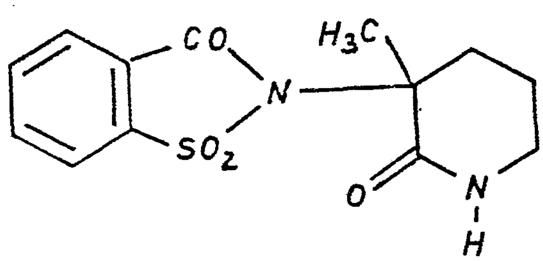

4) Neue Lactamverbindungen der Formel I gemäß Ansprüchen 1 - 3, dadurch gekennzeichnet, daß sie in optisch aktiver Form vorliegen.

5) Arzneimittel, dadurch gekennzeichnet, daß es als Wirkstoff eine Lactamverbindung gemäß Ansprüchen 1 - 4 enthält.

6) Arzneimittel nach Anspruch 5, dadurch gekennzeichnet, daß es als Wirkstoff pro Einzeldosis 50 bis 500 mg einer Lactamverbindung gemäß Ansprüchen 1 - 4 enthält.

7) Arzneimittel nach Ansprüchen 5 und 6, dadurch gekennzeichnet, daß es zur oralen Applikation bestimmt ist.

8) Arzneimittel nach Anspruch 7, dadurch gekennzeichnet, daß es in Form von Tabletten, Dragees oder Kapseln zur oralen Applikation vorliegt und pro Einzeldosis 50 bis 500 mg einer Lactamverbindung gemäß Ansprüchen 1 - 4 sowie übliche Hilfsstoffe und Trägermaterialien enthält.

9) Arzneimittel nach Ansprüchen 7 und 8, dadurch gekennzeichnet, daß es sich um Zubereitungen mit verzögerter Wirkstoffabgabe handelt, die eine Lactamverbindung gemäß Ansprüchen 1 - 4 enthalten.

10) Verfahren zur Herstellung von neuen Lactamverbindungen der Formel

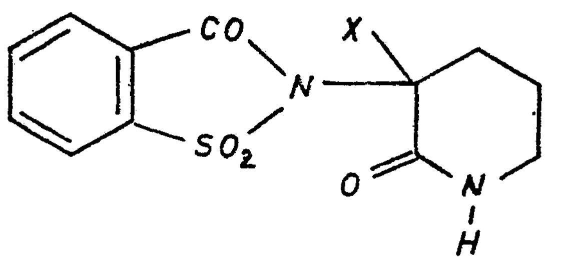

(I)

worin X für einen gegebenenfalls verzweigten Alkylrest mit 1 - 4 Kohlenstoffatomen steht,

dadurch gekennzeichnet, daß man

a) eine Verbindung der Formel

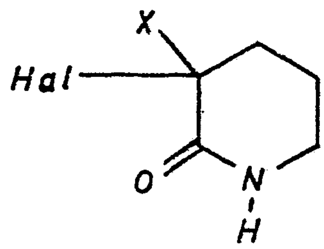

(II)

in der X die gleiche Bedeutung wie oben hat und Hal für ein Jodatom oder vorzugsweise für ein Chlor- oder Bromatom steht,

in Gegenwart eines Lösungs- oder Suspensionsmittels, gegebenenfalls unter Zusatz von Natrium- oder Kaliumjodid, vorzugsweise bei der Siedetemperatur des verwendeten Reaktionsmediums, mit einer Verbindung der Formel

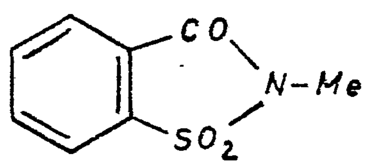

(III)

worin Me für ein Alkalimetall, vorzugsweise Natrium oder Kalium steht,

umsetzt, oder

b) eine Verbindung der allgemeinen Formel

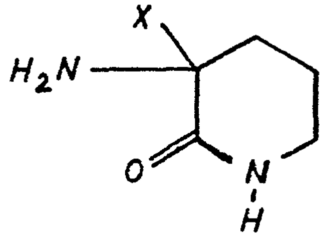

(IV)

worin X die gleiche Bedeutung wie oben hat

oder ein von einer Säure abgeleitetes Salz einer Verbindung der Formel IV in einem indifferenten Lösungsmittel mit o-Sulfobenzoesäuredichlorid oder -dibromid, gegebenenfalls unter Kühlung und vorzugsweise unter Zusatz von säurebindenden Stoffen, oder mit o-Sulfobenzoesäureanhydrid umsetzt, oder

c) eine Verbindung der allgemeinen Formel IV oder ein von einer Säure abgeleitetes Salz einer Verbindung der Formel IV in einem indifferenten Lösungsmittel bei Temperaturen von etwa $0^{o}$ - $100^{o}C$ und vorzugsweise in Gegenwart einer organischen Base mit einer Verbindung der Formel

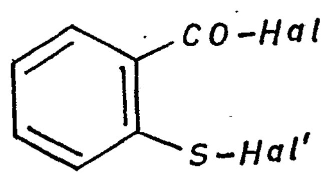

(V)

worin Hal und Hal' gleich oder verschieden sind und für ein Brom- oder Jodatom oder vorzugsweise für ein Chloratom stehen

umsetzt und anschließend die dabei erhaltene Verbindung der Formel

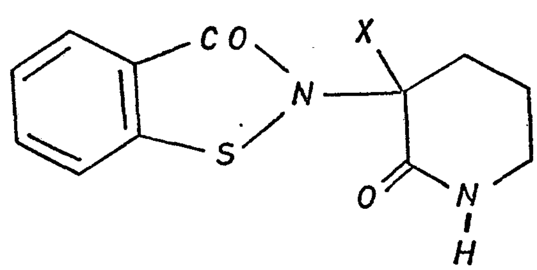

(VI)

in Gegenwart einer als Lösungsmittel dienenden aliphatischen Carbonsäure mit 1 bis 5 Kohlenstoffatomen bei 25 - $125^{o}C$ vorzugsweise mit Hilfe einer Peroxiverbindung zu einer Verbindung der Formel I oxydiert, oder

d) eine Verbindung der Formel

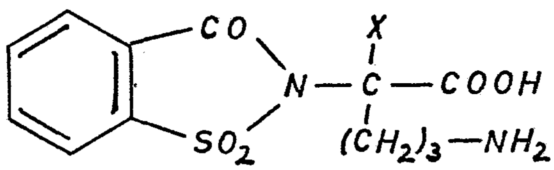

(VII)

worin X die gleiche Bedeutung wie oben hat,

oder ein funktionelles Derivat einer Säure der Formel VII durch Erhitzen, gegebenenfalls in Gegenwart eines hoch siedenden Lösungs- oder Suspensionsmittels, und/ oder durch Behandeln mit sauren, wasserabspaltend wirkenden Mitteln cyclisiert, oder

e) eine Verbindung der Formel

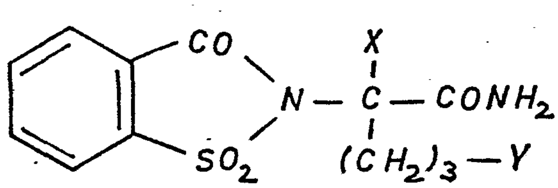

(VIII)

worin X die gleiche Bedeutung wie oben hat und Y für ein Chlor-, Brom- oder Jodatom oder für eine Hydroxylgruppe steht,

gegebenenfalls in Gegenwart eines indifferenten Lösungsmittels cyclisiert und zwar, wenn Y eines der genannten Halogenatome ist, in Gegenwart von organischen oder anorganischen Basen, oder, wenn Y eine Hydroxylgruppe bedeutet, in Gegenwart von sauren, wasserabspaltend wirkenden Mitteln oder durch Erhitzen, vorzugsweise in einem hochsiedenden Lösungs- oder Suspensionsmittel.

11) Verfahren zur Herstellung optisch aktiver Formen der Verbindungen der Formel I gemäß Anspruch 10, dadurch gekennzeichnet, daß man bei den Verfahrensvarianten a oder b von optisch aktiven Formen der Verbindungen der allgemeinen Formeln II oder IV ausgeht.

12) Verfahren zur Herstellung von Arzneimitteln, die als Wirkstoff eine Lactamverbindung gemäß Ansprüchen 1 - 4 enthalten, dadurch gekennzeichnet, daß man zur Herstellung von Tabletten, Dragees, Kapseln, Suppositorien, Sirupen, Säften oder Tropfen die Lactamverbindung mit üblichen Träger- und/oder Hilfsstoffen innig vermischt und dann die festen Formen zu solchen Einzeldosen verarbeitet, daß sie pro Einzeldosis 50 - 500 mg der Lactamverbindung enthalten.

13) Verwendung der neuen Lactamverbindungen gemäß Ansprüchen 1 - 4 zur Herstellung von Arzneimitteln mit das Zentralnervensystem dämpfenden Wirkungen.

14) Verwendung von 3-(2,3-Dihydro-1,1-dioxido-3-oxo-1,2-benzisothiazol-2-yl)-3-methyl-2-oxopiperidin und seinen optisch aktiven Formen zur Herstellung von Arzneimitteln mit sedierenden, antikonvulsiven und hypnonarkotischen Wirkungen.

Patentansprüche für Österreich

1) Verfahren zur Herstellung von neuen Lactamverbindungen der Formel

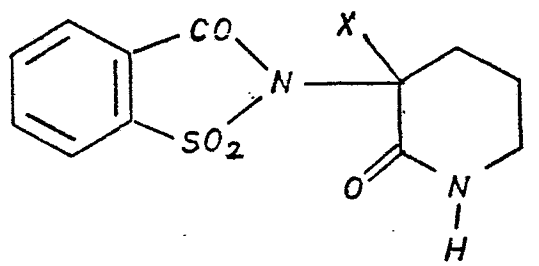

(I)

worin X für einen gegebenenfalls verzweigten Alkylrest mit 1 - 4 Kohlenstoffatomen steht,

dadurch gekennzeichnet, daß man

a) eine Verbindung der Formel

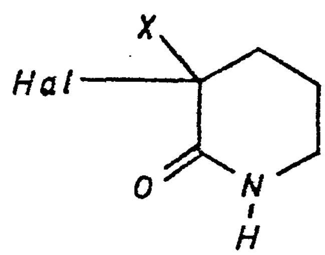

(II)

in der X die gleiche Bedeutung wie oben hat und Hal für ein Jodatom oder vorzugsweise für ein Chlor- oder Bromatom steht,

in Gegenwart eines Lösungs- oder Suspensionsmittels, gegebenenfalls unter Zusatz von Natrium- oder Kaliumjodid, vorzugsweise bei der Siedetemperatur des verwendeten Reaktionsmediums, mit einer Verbindung der

Formel

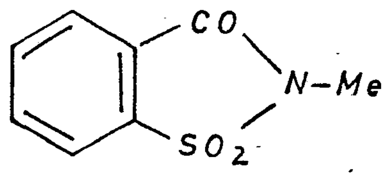

(III)

worin Me für ein Alkalimetall, vorzugsweise Natrium oder Kalium steht,

umsetzt, oder

b) eine Verbindung der allgemeinen Formel

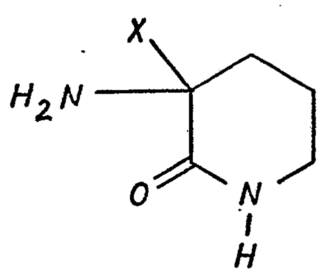

(IV)

worin X die gleiche Bedeutung wie oben hat

oder ein von einer Säure abgeleitetes Salz einer Verbindung der Formel IV in einem indifferenten Lösungsmittel mit o-Sulfobenzoesäuredichlorid oder -dibromid, gegebenenfalls unter Kühlung und vorzugsweise unter Zusatz von säurebindenden Stoffen, oder mit o-Sulfobenzoesäureanhydrid umsetzt, oder

c) eine Verbindung der allgemeinen Formel IV oder ein von einer Säure abgeleitetes Salz einer Verbindung der Formel IV in einem indifferenten Lösungsmittel bei Temperaturen von etwa $0^{o}$ - $100^{o}C$ und vorzugsweise in Gegenwart einer organischen Base mit einer Verbindung der Formel

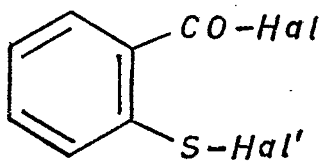

(V)

worin Hal und Hal' gleich oder verschieden sind und für ein Brom- oder Jodatom oder vorzugsweise für ein Chloratom stehen

umsetzt und anschließend die dabei erhaltene Verbindung der Formel

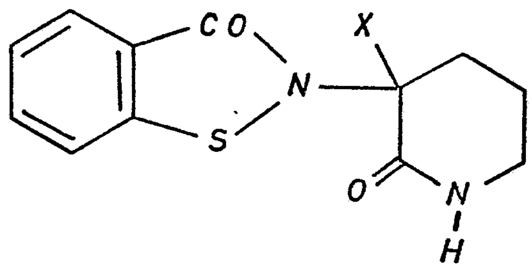

(VI)

in Gegenwart einer als Lösungsmittel dienenden aliphatischen Carbonsäure mit 1 bis 5 Kohlenstoffatomen bei 25 - 125°C vorzugsweise mit Hilfe einer Peroxiverbindung zu einer Verbindung der Formel I oxydiert, oder

d) eine Verbindung der Formel

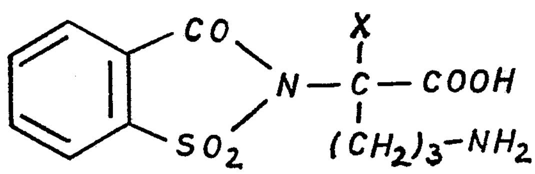

(VII)

worin X die gleiche Bedeutung wie oben hat,

oder ein funktionelles Derivat einer Säure der Formel VII durch Erhitzen, gegebenenfalls in Gegenwart eines hoch siedenden Lösungs- oder Suspensionsmittels, und/oder durch Behandeln mit sauren, wasserabspaltend wirkenden Mitteln cyclisiert, oder

e) eine Verbindung der Formel

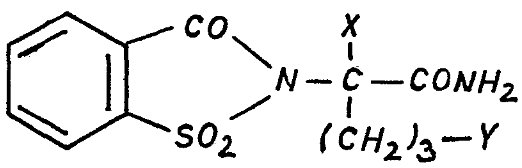

(VIII)

worin X die gleiche Bedeutung wie oben hat und Y für ein Chlor-, Brom- oder Jodatom oder für eine Hydroxylgruppe steht,

gegebenenfalls in Gegenwart eines indifferenten Lösungsmittels cyclisiert, und zwar, wenn Y eines der genannten Halogenatome ist, in Gegenwart von organischen oder anorganischen Basen, oder, wenn Y eine Hydroxylgruppe bedeutet, in Gegenwart von sauren, wasserabspaltend wirkenden Mitteln oder durch Erhitzen, vorzugsweise in einem hochsiedenden Lösungs- oder Suspensionsmittel.

2) Verfahren zur Herstellung optisch aktiver Formen der Verbindungen der Formel I gemäß Anspruch 1, dadurch gekennzeichnet, daß man bei den Verfahrensvarianten a oder b von optisch aktiven Formen der Verbindungen der allgemeinen Formeln II oder IV ausgeht.

Europäisches Patentamt

**EUROPÄISCHER RECHERCHENBERICHT**

0054672

Nummer der Anmeldung

EP 81 10 8664.4

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D,A | <u>DE - C3 - 2 210 166</u> (CHEMIE GRÜNENTHAL GMBH)<br>---- | | C 07 D 417/04<br>A 61 K 31/445 |
| | | | **RECHERCHIERTE SACHGEBIETE (Int. Cl.³)** |
| | | | A 61 K 31/00<br>C 07 D 417/04 |
| | | | **KATEGORIE DER GENANNTEN DOKUMENTE**<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 08-03-1982 | BREW |